(19) Europäisches Patentamt
European Patent Office
Office européen des brevets

(11) **EP 3 819 631 B1**

(12) **EUROPEAN PATENT SPECIFICATION**

(45) Date of publication and mention
of the grant of the patent:
**09.08.2023 Bulletin 2023/32**

(21) Application number: **20806137.4**

(22) Date of filing: **23.04.2020**

(51) International Patent Classification (IPC):
**G01N 1/38** *(2006.01)*   **G01N 21/80** *(2006.01)*
**G01N 21/85** *(2006.01)*   **G01N 33/18** *(2006.01)*
**G01N 35/00** *(2006.01)*   **G01N 21/01** *(2006.01)*
**G01N 21/05** *(2006.01)*   **G01N 1/10** *(2006.01)*

(52) Cooperative Patent Classification (CPC):
**G01N 21/80; G01N 1/38; G01N 21/85;**
**G01N 33/1893; G01N 35/00;** G01N 1/10;
G01N 21/05; G01N 2021/0193; G01N 2035/00465;
G01N 2201/0627; G01N 2201/08;
G01N 2201/12746

(86) International application number:
**PCT/CN2020/086451**

(87) International publication number:
**WO 2020/228507 (19.11.2020 Gazette 2020/47)**

(54) **HIGH-PRECISION SEAWATER PH IN-SITU MEASUREMENT SYSTEM AND METHOD BASED ON INTEGRATED VALVE-TERMINAL APPARATUS**

HOCHPRÄZISIONSSYSTEM UND -VERFAHREN ZUR IN-SITU-MESSUNG DES PH-WERTS VON MEERWASSER AUF DER GRUNDLAGE EINER INTEGRIERTEN VENTILINSEL

SYSTÈME ET PROCÉDÉ DE MESURE À HAUTE-PRÉCISION IN-SITU DU PH DE L'EAU DE MER FONDÉS SUR UN APPAREIL DE TERMINAL DE VANNES INTÉGRÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.05.2019 CN 201910404434**

(43) Date of publication of application:
**12.05.2021 Bulletin 2021/19**

(73) Proprietor: **Institute of Oceanographic Instrumentation,**
**Shandong Academy of Sciences**
**Qingdao, Shandong 266000 (CN)**

(72) Inventors:
• **CAO, Lu**
  **Qingdao, Shandong 266000 (CN)**
• **ZHANG, Yunyan**
  **Qingdao, Shandong 266000 (CN)**
• **ZHANG, Yingying**
  **Qingdao, Shandong 266000 (CN)**
• **ZHANG, Tianpeng**
  **Qingdao, Shandong 266000 (CN)**
• **CHU, Dongzhi**
  **Qingdao, Shandong 266000 (CN)**
• **WANG, Jingru**
  **Qingdao, Shandong 266000 (CN)**
• **WANG, Xiaohong**
  **Qingdao, Shandong 266000 (CN)**

(74) Representative: **Durán-Corretjer, S.L.P.**
**Còrsega, 329**
**(Paseo de Gracia/Diagonal)**
**08037 Barcelona (ES)**

(56) References cited:
CN-A- 101 603 969   CN-A- 103 439 258
CN-A- 103 439 258   CN-A- 103 698 287
CN-A- 103 698 287   CN-A- 104 122 217
CN-A- 105 067 542   CN-A- 105 241 829
CN-A- 105 241 829   CN-A- 110 057 814
CN-U- 210 071 669   CN-U- 210 071 669
US-A1- 2008 285 011

- **SPAULDING REGGIE S. ET AL: "Autonomous in Situ Measurements of Seawater Alkalinity", ENVIRONMENTAL SCIENCE & TECHNOLOGY, vol. 48, no. 16, 22 July 2014 (2014-07-22) , pages 9573-9581, XP055848976, US ISSN: 0013-936X, DOI: 10.1021/es501615x**
- **RÉROLLE VICTOIRE M.C. ET AL: "Seawater-pH measurements for ocean-acidification observations", TRAC TRENDS IN ANALYTICAL CHEMISTRY, vol. 40, 2012, pages 146-157, XP055848988, AMSTERDAM, NL ISSN: 0165-9936, DOI: 10.1016/j.trac.2012.07.016**

## Description

### Technical field

The invention belongs to the technical field of marine monitoring, and specifically relates to a high-precision seawater pH in-situ measurement system and a method based on an integrated valve terminal capable of applying to multiple indicators.

### Background

It is reported that seawater surface pH is continually declining at a rate of 0.002/a. The in-depth study of marine carbonate system puts forward further requirements for providing stable, reliable and high-precision pH measurement instruments. Currently the mainstream of sea water pH measurement has changed to a photometric method due to its advantages in high accuracy (up to 0.001) and good stability, especially for research on seawater acidification and carbon cycle. In regard to high-precision sea water pH in-situ measurement system, the photometric method is integrated with a flow injection analysis technology in which seawater pH could be obtained by measuring absorbance of indicators in varied forms into which samples are mixed.

Core technologies for constructing a high-precision seawater pH in-situ measurement system include flow path composition and control, selection of flow cell, light source coupling module, signal acquisition and detection and indicator calibration, which determines if a system is accurate and stable.

Typical in-situ pH sensor has a flow path comprising a three-way valve combination or a multi-position valve which has the disadvantages of large volume and high power consumption and the light source and signal detection mostly use a combination of LED and photodiode so merely one indicator could be measured which has a poor resolution and accuracy; those solutions may affect the measurement precision and range of the instrument.

CN 103698287 A discloses a low level light signal detection device for detecting the pH value of seawater comprising a flow path system including a valve group with four T-valves, a flow cell, an indicator container, a peristaltic indicator pump, and a peristaltic seawater sampling pump, a light path detection system and a main control system.

CN 105241829 A discloses a high precision seawater pH measurement mechanism comprising fluid communication means and signal supervisory instrument, the fluid communication means comprising an indicator bag, a first, seconds and third T-valves, a flow cell, a peristaltic pump, a pulse pump and a fluid circuit.

CN 103439258 A discloses an *in situ* seawater chemical analysis system based on integral valve termina device comprising an integrated valve terminal, a peristaltic pump, a well heater, an optical flow cell, LED lamp, photoelectricity acquisition module and a computer processing module.

SPAULDING REGGIE S. ET AL, "Autonomous in Situ Measurements of Seawater Alkalinity", ENVIRONMENTAL SCIENCE & TECHNOLOGY, US, (20140722), vol. 48, no. 16, doi:10.1021/es501615x, ISSN 0013-936X, pages 9573 - 9581, discloses an evaluation work of the performance of an autonomous instrument for high temporal resolution measurements of seawater total alkalinity.

RÉROLLE VICTOIRE M.C. ET AL, "Seawater-pH measurements for ocean-acidification observations", TRAC TRENDS IN ANALYTICAL CHEMISTRY, AMSTERDAM, NL, (2012), vol. 40, doi:10.1016/j.trac.2012.07.016, ISSN 0165-9936, pages 146 - 157, discloses Seawater-pH measurements for ocean-acidification observations wherein a simple microfluidic design integrated in a shipboard instrument is presented featuring high accuracy and precision as a key step towards a targeted pH microsensor system.

### Summary

In view of the above technical problems, the present invention provides a high-precision seawater pH in-situ measurement system and method based on an valve terminal that can detect multiple indicators; the flow path structure of the high-precision seawater pH in-situ measurement system is based on the valve terminal device, which is small in dead area and good in stability; and the optical path structure uses LED coupled light source and micro spectrometer as light source and detector, which can use a variety of indicators for measurement, which enlarges the measurement range and improves measurement accuracy.

The present invention is directed to a high-precision seawater pH in-situ measurement system as defined in claim 1 and to a high-precision seawater pH in-situ measurement method using said system as defined in claim 4. Preferred embodiments of the invention are defined in the dependent claims.

A high-precision seawater pH in-situ measurement system based on valve terminal includes: a flow path module, an optical path module and a circuit module; wherein;

the flow path module including a valve terminal, a flow cell, and an indicator bag connected to the valve terminal through a pulse pump, wherein one end of the flow cell is connected to the valve terminal through a peristaltic pump, and the other end of the flow cell is connected to the valve terminal; the valve terminal is further provided with a seawater sampling pipeline, a seawater waste liquid discharge pipeline and an indicator waste liquid discharge pipeline;

the optical path module is used to measure absorbance of sample at characteristic wavelengths, which includes a light source coupling module, a miniature spectrometer and optical fibers; wherein the light source coupling module includes three LEDs of different wavelengths arranged therein; the light source coupling module with three LEDs emits light, and the light is transmitted through the optical fibers and arrives at the miniature spectrometer, and the miniature spectrometer is configured to detect intensity of optical signal after passing through the flow cell;

the circuit module is respectively connected to the peristaltic pump, the pulse pump, the valve terminal and the spectrometer; the circuit module is configured to control the on-off of the peristaltic pump, the pulse pump, the valve terminal and the miniature spectrometer and further used for data collection.

Further, the valve terminal includes four two-position three-way solenoid valves: a first solenoid valve, a second solenoid valve, a third solenoid valve, and a fourth solenoid valve; each of the solenoid valve has a total of three ports: a normally closed port *a,* a normally open port *b* and a third port;

the seawater sampling pipeline is connected to the normally open port *b* of the first solenoid valve, and

the third port of the first solenoid valve is connected to the normally open port *b* of the second solenoid valve; the normally closed port *a* of the second solenoid valve is connected to the indicator bag through the pulse pump, the third port of the second solenoid valve is connected to the third port of the third solenoid valve, and the normally open port *b* of the third solenoid valve is connected to one end of the flow cell through the peristaltic pump, the normally closed port a of the third solenoid valve is connected to the indicator waste liquid discharge pipeline, and the normally closed port a of the fourth solenoid valve is connected to the normally closed port *a* of the first solenoid valve, the normally open port *b* of the fourth solenoid valve is connected to the seawater waste liquid discharge pipeline, and the third port of the fourth solenoid valve is connected to the other end of the flow cell.

Preferably, the valve terminal includes a base plate and a bottom plate, wherein the bottom plate is arranged below the base plate and the four solenoid valves are arranged below the bottom plate;

the base plate and the bottom plate are made of acrylic material with high grade of transparency, and are seamlessly bonded by adhesive bonding in a dust-free and clean environment condition; each of the base plate and the bottom plate is respectively provided with liquid channels therein configured to communicate the solenoid valves with external fluidic pipelines connected to the valve terminal;

the external fluidic pipelines connected to the valve terminal include the fluidic pipeline between the peristaltic pump and the valve terminal, the fluidic pipeline between the pulse pump and the valve terminal, the indicator waste liquid discharge pipeline, the seawater waste liquid discharge pipeline, the seawater sampling pipeline, the pipeline between the flow cell and the valve terminal and the pipeline between the third port of the first solenoid valve and the normally open port *b* of the second solenoid valve;

the liquid channels are further disposed between the first solenoid valve and the fourth solenoid valve, and configured to connect the second solenoid valve to the third solenoid valve;

the inner diameter of the liquid channels inside the base plate and the bottom plate is the same as the inner diameter of the external fluidic pipelines connected to the valve terminal.

Preferably, the flow cell is made of plastic material and has a cross-shaped structure wherein the fluid flows from bottom to top, and the light signal is transmitted in a horizontal direction; air bubbles generated in the fluid could float to the top of the flow cell to avoid the influence of the accumulation of air bubbles on absorbance measurement; the light path length of the flow cell is 0.5-4 cm, and the flow cell is placed in seawater outside an instrument compartment to keep the temperature of the sample in the flow cell consistent; a temperature probe is also set next to the flow cell for real-time measurement of the seawater temperature.

Preferably, the three LEDs of different wavelengths include: a warm white LED with a center wavelength of 580 nm and a wider peak width, an LED with a center wavelength of 435 nm and an LED with a center wavelength of 490 nm;
the spectrum detection range of the spectrometer is 300 nm - 800 nm, equipped with a 25 $\mu$m slit, and the wavelength resolution is 1.5 nm; the miniature spectrometer is a miniature spectrometer on the market, with small volume and power consumption, and is suitable for in-situ pH sensors.

The arrangement of the coupled light source with three LEDs of different wavelengths and the spectrometer is applied to the seawater pH measurement with three different indicators: *m-cresol* purple, *thymol* blue and *cresol* red and the capable pH measurement range is 6.8-8.6.

Preferably, the flow path is controlled by the valve terminal; when performing an in-situ seawater pH measurement, the seawater is first injected into the flow path to determine a background light intensity, and then the indicator is injected into the flow path; the flow path is closed into a loop, the seawater and the indicator are mixed within the loop and then the light intensity of the mixed solution is detected for the calculation of the absorbance, and the seawater pH is obtained on the basis of the absorbance.

A high-precision seawater pH in-situ measurement method based on valve terminal using the measurement system includes:

seawater sampling: switching the first solenoid valve, the second solenoid valve, the third solenoid valve and the fourth solenoid valve to the normally open port *b*; turning on the peristaltic pump to guide a seawater sample to enter and fill with the flow path from the seawater sampling pipeline; wherein excess seawater is discharged via the seawater waste liquid discharge pipeline;

background light intensity detection: turning off the peristaltic pump; turning on LEDs; measuring a background light intensity of the seawater sample without being added indicator into by the spectrometer at characteristic wavelengths of selected indicators, wherein the characteristic wavelengths of selected indicators include a wavelength at a maximum absorption point in an acid state, a wavelength at a maximum absorption point in an alkali state, and a wavelength at an isobestic point in an acid state and in an alkali state; wherein the optical signal detected by the spectrometer is expressed as $I_{0\lambda}$ and $\lambda$ indicating the wavelength;

taking a selected indicator *thymol* blue as an example, the wavelength at the maximum absorption point in an acid state is 435 nm, the wavelength at the maximum absorption point in an alkali state is 596 nm, and the wavelength at the isobestic point in an acid state and in an alkali state is 494 nm, and accordingly, the optical signal detected by the spectrometer is respectively expressed as $I_{0\lambda435}$ , $I_{0\lambda596}$, $I_{0\lambda494}$.

Adding indicator: switching the second solenoid valve 5 to the normally closed port *a*; switching the third solenoid valve 6 to the normally closed port *a*; turning on the pulse pump 2 to draw a selected indicator into an indicator flow path from the indicator bag, wherein excess indicator is discharged through the indicator waste liquid discharge pipeline 10; turning off the pulse pump.

Sample mixing: switching the second solenoid valve 5 to the normally open port *b*; switching the third solenoid valve 6 to the normally open port *b*; switching the first solenoid valve 4 to the normally closed port *a*; switching the fourth solenoid valve 7 to the normally closed port a; forming a closed loop; forming a circulation system where the valve terminal, the flow cell and pipelines are connected; turning on the peristaltic pump to fully mix the seawater sample and the indicator and the time for mixing is 20-180 s which could be set according to speed range of the pulse pump and the pipelines used; turning off the peristaltic pump when the seawater sample and the indicators are fully mixed;
detecting light intensity of the mixed solution and computing absorbance: turning on LEDs when the seawater sample and the indicators are fully mixed; measuring light intensity of the mixed solution wherein the optical signal detected is expressed by $I_\lambda$, computing absorbance by $A_\lambda = -\log_{10} (I_\lambda/I_{0\lambda})$; computing pH according to the measurement result of the spectrometer.

Preferably, the method for calculating the pH of seawater is specifically as follows: referring to the photometric method, the pH of seawater is determined by the secondary dissociation equilibrium reaction of the acid-base indicator. The calculation formula is:

$$pH_T = pK_2^T + \log_{10} \frac{[I^{2-}]}{[HI^-]} ,$$

$pH_T$ represents the pH under the scale of total hydrogen ion, $K_2^T$ is the secondary dissociation constant of the indicator; $[I^{2-}]$ and $[HI^-]$ are the concentrations of the indicator in the alkali state and in the acid state;

measuring the absorbance $A_{\lambda_1}$ of the sampling at the wavelength $\lambda_1$ of the maximum absorption point of [HI⁻] and the absorbance $A_{\lambda_2}$ of the sampling at the wavelength $\lambda_2$ of the maximum absorption point of [I²⁻] respectively; according to Lambert Beer's law, the calculation formula could be written as:

$$\mathrm{pH}_T = -\log_{10}(K_2^T) + \log_{10}\left(\frac{R - e_1}{e_2 - R \times e_3}\right)$$

wherein $K_2^T$ determines the measurement range of the indicator, and the pH measurement of seawater could be carried out by using the applicable seawater pH measurement indicator; wherein

$$e_1 = \frac{\varepsilon_{HI^-\lambda_2}}{\varepsilon_{HI^-\lambda_1}}, \qquad e_2 = \frac{\varepsilon_{I^2-\lambda_2}}{\varepsilon_{HI^-\lambda_1}}, \qquad e_3 = \frac{\varepsilon_{I^2-\lambda_1}}{\varepsilon_{HI^-\lambda_1}}, \qquad R = \frac{A_{\lambda_2}}{A_{\lambda_1}}$$

wherein $e_1$, $e_2$ and $e_3$ are ratios of molar absorption coefficients at different forms and different wavelengths of the indicator which are related to temperature and salinity; R is the ratio of absorbance; $\varepsilon_{I2-\lambda_1}$ and $\varepsilon_{I2-\lambda_2}$ are the absorbance coefficients of I²⁻ at wavelengths $\lambda_1$ and $\lambda_2$; $\varepsilon_{HI^-\lambda_1}$ and $\varepsilon_{HI^-\lambda_2}$ are the absorbance coefficients of HI⁻ at $\lambda_1$ and $\lambda_2$.

Preferably, the method includes a step for indicator correction: switching the first solenoid valve to the normally open port b; switching the fourth solenoid valve to the normally open port *b* when the step of detecting light intensity of the mixed solution and computing absorbance is finished; turning on the peristaltic pump to guide a seawater sample into pipelines to dilute the indicator; continuously measuring a plurality of light intensity of the mixed solution during the course of dilution by the spectrometer and calculating corresponding absorbance and pH, wherein the time required for dilution could be set as 20 to 180 s; due to the fact that the absorbance of the indicator at the isobestic point in the acid state and in the alkali state is merely related to the concentration of the indicator, a rectangular coordinate system is established where the abscissa indicates the absorbance, the ordinate indicates the pH and the measured absorbance and the calculated pH in one-to-one correspondence is denoted as a coordinate point; for a coordinate point where x=0, y indicates the corrected seawater pH where the interference by the indicator is eliminated.

The beneficial technical effects of the present invention:

The flow path structure of the high-precision seawater pH in-situ measurement system provided by the present invention is based on an valve terminal, has the characteristics of small dead volume, compact structure, and low power consumption, can realize complete mixing of seawater and indicator, and can improve instrument measurement stability.
The optical path structure of the high-precision seawater pH in-situ measurement system provided by the present invention uses an LED coupled light source and a miniature spectrometer as a light source and a detector, and can use a variety of indicators for measurement, broadens the measurement range and improves the measurement accuracy.

**Brief Description of the Drawings**

Fig. 1 shows the structure and the flow path of the high-precision seawater pH in-situ measurement system based on valve terminal according to one aspect of the present invention;
Fig. 2 is a three-dimensional schematic diagram of a valve terminal of a system according to one aspect of the present invention;

wherein 1.indicator bag, 2.pulse pump, 3.valve terminal, 4.first solenoid valve, 5.second solenoid valve, 6.third solenoid valve, 7.fourth solenoid valve, 8.seawater sampling pipeline, 9.seawater waste liquid discharge pipeline,10.indicator waste liquid discharge pipeline, 11.peristaltic pump, 12.light source coupling module; 13.flow cell; 14.miniature spectrometer; 3-1. base plate; 3-2. bottom plate; 3-3. normally open port b; 3-4. third port; 3-5. normally closed port a; 3-6. hole with smooth inner wall; 3-7. fixing screw hole; 3-8. threaded hole; 3-9. base plate liquid channel; 3-10. bottom plate liquid channel; 3-11. valve terminal internal liquid channel.

## Detailed Description

In order to make the objectives, technical solutions and advantages of the present invention clear, the following further describes the present invention in detail with reference to the accompanying drawings and embodiments. It should be understood that the specific embodiments described here are only used to explain the present invention, but not to limit the present invention.

The scope of the invention is defined by the appended claims.

Further, in order to enable the public to have a better understanding of the present invention, in the following detailed description of the present invention, some specific details are described in detail. Those skilled in the art can fully understand the present invention without the description of these details.

As described in the background, typical in-situ pH sensor has a flow path comprising a three-way valve combination, a multi-position valve and the like, which has the disadvantages of large volume and high power consumption, in which the light source and signal detection mostly use a combination of LED and photodiode so merely one indicator could be measured and has a poor performance in resolution and accuracy; those techniques may affect the measurement precision and range of the instrument. In order to make improvements, a high-precision seawater pH in-situ measurement system based on a valve terminal capable of applying to multiple indicators is provided which is on the basis of the photometric measurement and circulation analysis to obtain pH value by measured change of absorbance of sample in which indicators and seawater are completely mixed.

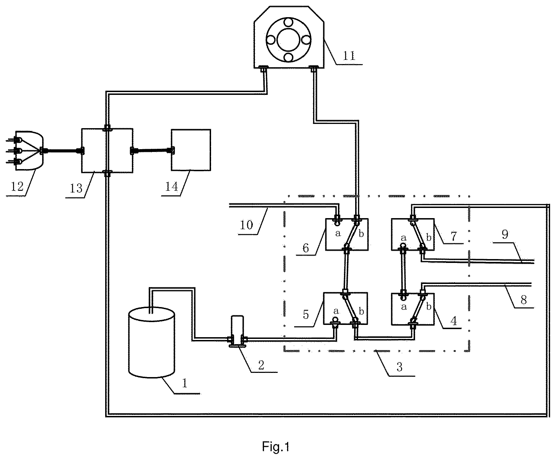

As shown in Fig.1, the measurement system includes: a flow path module, an optical path module and a circuit module.

The flow path module includes a valve terminal, a flow cell, and an indicator bag connected to the valve terminal through a pulse pump; one end of the flow cell is connected to the valve terminal through a peristaltic pump, and the other end of the flow cell is connected to the valve terminal; the valve terminal is also provided with a seawater sampling pipeline, a seawater waste liquid discharge pipeline and an indicator waste liquid discharge pipeline; wherein the flow path module also includes fluidic pipelines: the indicator bag and the pulse pump are connected by a fluidic pipeline, the pulse pump and the valve terminal are connected by a fluidic pipeline, the peristaltic pump and the valve terminal are connected by a fluidic pipeline, the peristaltic pump and the flow cell are connected by a fluidic pipeline, and the flow cell and the valve terminal are connected by a fluidic pipeline.

The optical path module is used to measure absorbance of sample at characteristic wavelengths, which includes a light source coupling module, a miniature spectrometer and optical fibers; wherein the light source coupling module includes three LEDs of different wavelengths arranged therein. The light source coupling module with three LEDs emits light, and the light is transmitted through the optical fibers and finally arrives at the miniature spectrometer, and the miniature spectrometer is configured to detect intensity of optical signal after passing through the flow cell.

The circuit module is respectively connected to the peristaltic pump, the pulse pump, the valve terminal and the spectrometer; the circuit module is configured to control the on-off of the peristaltic pump, the pulse pump, the valve terminal and the miniature spectrometer and also used for data collection. Preferably, the circuit system adopts a modular design concept. During measurement, corresponding circuit modules are turned on or turned off according to preset working flows; with the arrangement the static working power consumption could be reduced. Taking consideration of the working environment of the measurement system, a leakage detection circuit is provided which is configured to switch electricity off if faults in electrical currents being detected, so as to protect components of the measurement system and to upload of default warning information.

In the present embodiment, the valve terminal includes four two-position three-way solenoid valves: a first solenoid valve, a second solenoid valve, a third solenoid valve, and a fourth solenoid valve; each of the solenoid valve has a total of three ports: a normally closed port a, a normally open port b and a third port.

The seawater sampling pipeline is connected to the normally open port *b* of the first solenoid valve, and the third port of the first solenoid valve is connected to the normally open port *b* of the second solenoid valve. The normally closed port *a* of the second solenoid valve is connected to the indicator bag through the pulse pump, the third port of the second solenoid valve is connected to the third port of the third solenoid valve, and the normally open port *b* of the third solenoid valve is connected to one end of the flow cell through the peristaltic pump, the normally closed port *a* of the third solenoid valve is connected to the indicator waste liquid discharge pipeline, and the normally closed port a of the fourth solenoid valve is connected to the normally closed port *a* of the first solenoid valve, the normally open port *b* of the fourth solenoid valve is connected to the seawater waste liquid discharge pipeline, and the third port of the fourth solenoid valve is connected to the other end of the flow cell.

In the present embodiment, the valve terminal includes a base plate and a bottom plate, wherein the bottom plate is arranged below the base plate and the four solenoid valves are arranged below the bottom plate.

The base plate and the bottom plate are made of acrylic material with high grade of transparency, and are seamlessly bonded by adhesive bonding in a dust-free and clean environment condition; each of the base plate and the bottom plate is respectively provided with liquid channels therein configured to communicate the solenoid valves with external fluidic pipelines connected to the valve terminal.

The external fluidic pipelines connected to the valve terminal include the fluidic pipeline between the peristaltic pump and the valve terminal, the fluidic pipeline between the pulse pump and the valve terminal, the indicator waste liquid discharge pipeline, the seawater waste liquid discharge pipeline, the seawater sampling pipeline, the pipeline between the flow cell and the valve terminal and the pipeline between the third port of the first solenoid valve and the normally open port *b* of the second solenoid valve.

The liquid channels are further disposed between the first solenoid valve and the fourth solenoid valve, and configured to connect the second solenoid valve to the third solenoid valve.

The inner diameter of the liquid channels inside the base plate and the bottom plate is the same as the inner diameter of the external fluidic pipelines connected to the valve terminal, so as to prevent fluctuation of flow rate when fluid flowing into the valve terminal and ensure measurement accuracy. In the present embodiment, the inner diameter of the liquid channel inside the base plate and the bottom plate and the inner diameter of the external fluidic pipelines connected to the valve terminal are set as 0.1-3 mm.

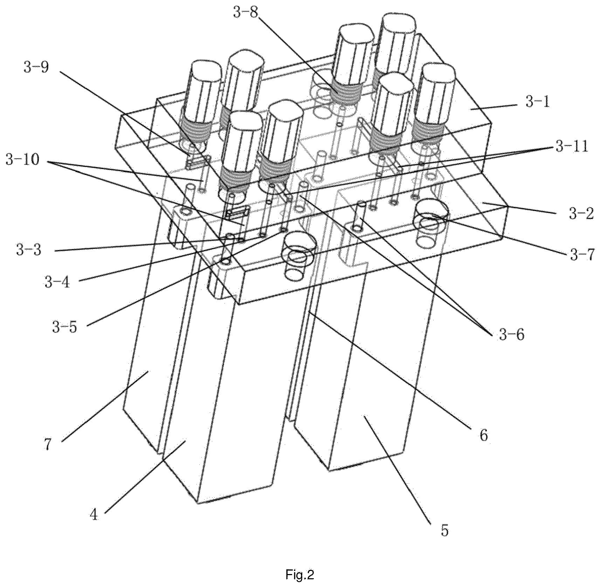

Fig.2 shows a specific structure of the valve terminal.

The liquid channels provided inside the base plate and the bottom plate include bottom plate liquid channels 3-10, base plate liquid channels 3-9, and valve terminal internal liquid channels 3-11.

The bottom plate is thoroughly provided with bottom plate liquid channels which are communicated with the solenoid valves respectively; to be specific three bottom plate liquid channels are thoroughly provided on the bottom plate which are respectively connected to the normally open port (shown as b3-3), the third port (shown as 3-4) and the normally closed port (shown as a3-5) of each solenoid valve, that is to say, a total of 12 bottom plate liquid channels are disposed on the valve terminal.

An upper part of the base plate is opened with threaded holes 3-8, the fluidic pipeline between the peristaltic pump and the valve terminal, the fluidic pipeline between the pulse pump and the valve terminal, and the indicator waste liquid discharge pipeline, the seawater waste liquid discharge pipeline, the seawater sampling pipeline, the fluidic pipeline between the flow cell and the valve terminal, and the pipeline between the third port of the first solenoid valve and the normally open port *b* of the second solenoid valve are connected to the threaded holes on the base plate of the valve terminal, and in the present embodiment, there are 8 threaded holes opened on the base plate.

The base plate liquid channels 3-9 are provided at a lower part of the base plate and the base plate liquid channels correspond to the threaded holes 3-8 one-to-one, and there are also 8 base plate liquid channels are provided; upper end of each base plate liquid channel is in communication with a threaded hole and a lower end of each base plate liquid channel is in communication with a corresponding bottom plate liquid channel 3-10, thereby connecting the external fluidic pipelines of the valve terminal and the solenoid valves; each threaded hole is provided with a joint for clamping and fixing a fluidic pipeline; specifically, the threaded hole is a flat-bottomed threaded hole.

The lower end surface of the base plate and the upper end surface of the bottom plate are simultaneously grooved and bonded to form the valve terminal internal liquid channels 3-11, and the valve terminal internal liquid channels include a first internal liquid channel and a second internal liquid channel.

Two ends of the first internal liquid channel are respectively connected to a bottom plate liquid channel corresponding to the third port of the second solenoid valve and to a bottom plate liquid channel corresponding to the third port of the third solenoid valve so as to connect the third port of the second solenoid valve to the third port of the third solenoid valve; two ends of the second internal liquid channel are respectively connected to a bottom plate liquid channel corresponding to the normally closed port *a* of the fourth solenoid valve and to a bottom plate liquid channel corresponding to the normally closed port *a* of the first solenoid valve so as to connect the normally closed port a of the fourth solenoid valve to the normally closed port *a* of the first solenoid valve.

Specifically, 4 sets of holes 3-6 with smooth inner walls for installing and fixing four solenoid valves are provided at the lower end of the bottom plate; fixing screw holes 3-7 for fixing the valve terminal are also provided at the lower end of the bottom plate. The solenoid valve could be a typical two-position three-way valve made of PEEK preventing from corrosion caused by high salinity, acidity or alkalinity. Fluids are driven by a peristaltic pump or pulse pump to enter the liquid channels in the valve terminal through the external fluidic pipelines connected thereto and then enter into the solenoid valves, thereby controlling the fluid flow direction.

The valve terminal makes the flow path system compact and reduces the dead volume of the flow path and the power consumption of the system.

In the present embodiment, the flow cell is made of plastic material and has a cross-shaped structure. The fluid flows from bottom to top, and the light signal is transmitted in a horizontal direction. Air bubbles generated in the fluid could float to the top of the flow cell to avoid the influence of the accumulation of air bubbles on absorbance measurement.

The light path length of the flow cell is 0.5-4 cm, and the flow cell is placed in seawater outside an instrument compartment to keep the temperature of the sample in the flow cell consistent; a temperature probe is also set next to the flow cell for real-time measurement of the seawater temperature.

In the present embodiment, the three LEDs of different wavelengths include: a warm white LED with a center wavelength of 580 nm and a wider peak width, an LED with a center wavelength of 435 nm and an LED with a center wavelength of 490 nm.

The spectrum detection range of the spectrometer is 300 nm - 800 nm, equipped with a 25 $\mu$m slit, and the wavelength resolution is 1.5 nm.

The arrangement of the coupled light source with three LEDs of different wavelengths and the spectrometer could be applied to the seawater pH measurement with three different indicators: *m-cresol* purple, *thymol* blue and *cresol* red and the capable pH measurement range is 6.8-8.6.

In the present embodiment, the selection of the LEDs depends on the characteristic wavelengths of the indicator used, including a wavelength at the maximum absorption point in an acid state, a wavelength at the maximum absorption point in an alkali state, and a wavelength at the isosbestic point in the acid state and the alkali state. At present, the commonly used indicators in seawater pH measurement include *m-cresol* purple, *thymol* blue and *cresol* red. The three indicators have a wavelength of 430-440 nm at the maximum absorption point in the acid state, a wavelength of 550-600 nm at the maximum absorption point in the acid state, and a wavelength of mostly in a range of 480-500 nm varied with temperature at the isosbestic point in the acid state and the alkali state. Therefore, in the present invention, using the above three LEDs of different wavelengths to form a coupled light source could meet the measurement requirements of the three indicators of *m-cresol* purple, *thymol* blue and *cresol* red, and could fulfill seawater pH measurement by the three indicators.

In addition, in the present embodiment, a small size and low power consumption miniature spectrometer is selected as a signal detection unit, the spectrum detection range is 300 nm - 800 nm, it is equipped with a 25 $\mu$m slit, and the wavelength resolution is 1.5 nm. If the wavelength resolution of the signal detection unit selected by the measurement system is higher than 2 nm, seawater pH could be calculated according to the published related data of the indicators. If the wavelength resolution of the signal detection unit selected by the measurement system is lower than 2 nm, in order to ensure the measurement accuracy, it is needed to determine the characteristic coefficients of the indicators. The miniature spectrometer could accurately measure the absorbance at the characteristic wavelengths of the indicators, and could measure the absorbance at multiple wavelengths at the same time. It is not interfered by other circuits during working and has stable performance. It further could meet the requirements of high-precision measurement by multiple indicators to broaden the application of the measurement system.

The pH range capable of being tested by an indicator is mainly determined by a second-order dissociation constant K2, and is also affected by a molar absorption coefficient. The best measurement range is $PK_2-1 \leq pH \leq pK_2$. Commonly used indicators for measuring seawater pH are *m-cresol* purple, *thymol* blue and *cresol* red. According to the prior art, the measuring range of *m-cresol* purple is 7.2-8.2, and the measuring range of *thymol* blue is 7.6-8.6 and the measurement range of phenol red is 6.8-7.8. According to the current seawater pH distribution, *m-cresol* purple is suitable for section and offshore seawater, *thymol* blue is suitable for ocean surface seawater, and *cresol* red is suitable for seawater in low pH areas.

In the present embodiment, the flow path is controlled by the valve terminal. When performing an in-situ seawater pH measurement, the seawater is first injected into the flow path to determine a background light intensity, and then the indicator is injected into the flow path. The flow path is closed into a loop, the seawater and the indicator are mixed within the loop and then the light intensity of the mixed solution is detected for the calculation of the absorbance, and the seawater pH could be obtained on the basis of the absorbance.

Among them, the structure of the closed flow path has the characteristics of small dead volume, compact structure, and low power consumption, which can fully mix seawater and indicators to improve the stability of measurement. In the present embodiment, the fluidic pipeline is made of polytetrafluoroethylene with an inner diameter of 0.1-3 mm; the indicator bag is made of aluminum foil material, which is light-proof and airtight; the indicator concentration is 0.1-5 mmol/L; the pulse pump is a micro pump with a pulse volume of 10-500 $\mu$L.

Another aspect of the present invention provides a high-precision seawater pH in-situ measurement method based on the measurement system of the above-mentioned embodiment, which is capable of applying to a plurality of indicators. The method includes following steps.

Seawater sampling: switching the first solenoid valve 4, the second solenoid valve 5, the third solenoid valve 6 and the fourth solenoid valve 7 to the normally open port *b*; turning on the peristaltic pump to guide a seawater sample to enter and fill with the flow path from the seawater sampling pipeline 8; wherein excess seawater is discharged via the seawater waste liquid discharge pipeline 9.

Background light intensity detection: turning off the peristaltic pump; turning on LEDs; measuring a background light intensity of the seawater sample without being added indicator into by the spectrometer at characteristic wavelengths of selected indicators, wherein the characteristic wavelengths of selected indicators include a wavelength at a maximum absorption point in an acid state, a wavelength at a maximum absorption point in an alkali state, and a wavelength at an isosbestic point in an acid state and in an alkali state; wherein the optical signal detected by the spectrometer is expressed as $\lambda I_0$ and $\lambda$ indicating the wavelength.

Taking a selected indicator *thymol* blue as an example, the wavelength at the maximum absorption point in an acid state is 435 nm, the wavelength at the maximum absorption point in an alkali state is 596 nm, and the wavelength at the isobestic point in an acid state and in an alkali state is 494 nm, and accordingly, the optical signal detected by the spectrometer is respectively expressed as $I_{0\lambda435}$ , $I_{0\lambda596}$, $I_{0\lambda494}$.

Adding indicator: switching the second solenoid valve 5 to the normally closed port a; switching the third solenoid valve 6 to the normally closed port a; turning on the pulse pump 2 to draw a selected indicator into an indicator flow path from the indicator bag, wherein excess indicator is discharged through the indicator waste liquid discharge pipeline 10; turning off the pulse pump.

Sample mixing: switching the second solenoid valve 5 to the normally open port b; switching the third solenoid valve 6 to the normally open port b; switching the first solenoid valve 4 to the normally closed port a; switching the fourth solenoid valve 7 to the normally closed port a; forming a closed loop; forming a circulation system where the valve terminal, the flow cell and pipelines are connected; turning on the peristaltic pump to fully mix the seawater sample and the indicator and the time for mixing is 20-180 s which could be set according to speed range of the pulse pump and the pipelines used; turning off the peristaltic pump when the seawater sample and the indicators are fully mixed.

Detecting light intensity of the mixed solution and computing absorbance: turning on LEDs when the seawater sample and the indicators are fully mixed; measuring light intensity of the mixed solution wherein the optical signal detected is expressed by $I_\lambda$, computing absorbance by $A_\lambda = -\log_{10} (I_\lambda/I_0^\lambda)$. The spectrometer selected could measure light intensity within the wavelength range of 300-800 nm which covers the entire measurement range merely needed to select a desired output wavelength. If the indicator is changed, the desired output wavelength should be changed as well.

Computing pH according to the measurement result of the spectrometer:

Referring to the photometric method, the pH of seawater is determined by the secondary dissociation equilibrium reaction of the acid-base indicator. The calculation formula is:

$$pH_T = pK_2^T + \log_{10} \frac{[I^{2-}]}{[HI^-]} ,$$

$pH_T$ represents the pH under the scale of total hydrogen ion, $K_2^T$ is the secondary dissociation constant of the indicator, $[I^{2-}]$ and $[HI^-]$ are the concentrations of the indicator in the alkali state and in the acid state.

Measuring the absorbance $A_{\lambda1}$ of the sampling at the wavelength $\lambda_1$ of the maximum absorption point of $[HI^-]$ and the absorbance $A_{\lambda2}$ of the sampling at the wavelength $\lambda_2$ of the maximum absorption point of $[I^{2-}]$ respectively.

According to Lambert Beer's law, the calculation formula could be written as:

$$pH_T = -\log_{10}(K_2^T) + \log_{10}(\frac{R - e_1}{e_2 - R \times e_3}) ,$$

wherein $K_2^T$ determines the measurement range of the indicator, and the pH measurement of seawater could be carried out by using the applicable seawater pH measurement indicator; wherein:

$$e_1 = \frac{\varepsilon_{HI^-\lambda_2}}{\varepsilon_{HI^-\lambda_1}} , \qquad e_2 = \frac{\varepsilon_{I^{2-}\lambda_2}}{\varepsilon_{HI^-\lambda_1}} , \qquad e_3 = \frac{\varepsilon_{I^{2-}\lambda_1}}{\varepsilon_{HI^-\lambda_1}} , \qquad R = \frac{A_{\lambda_2}}{A_{\lambda_1}}$$

wherein $e_1$, $e_2$ and $e_3$ are ratios of molar absorption coefficients at different forms and different wavelengths of the indicator; R is the ratio of absorbance; $\varepsilon_{I2-\lambda_1}$ and $\varepsilon_{I2-\lambda_2}$ are the absorbance coefficients of $I^{2-}$ at wavelengths $\lambda_1$ and $\lambda_2$; $\varepsilon_{HI^-\lambda_1}$ and $\varepsilon_{HI^-\lambda_2}$ are the absorbance coefficients of HI' at $\lambda_1$ and $\lambda_2$, which are related to temperature and salinity.
In the present embodiment, the method further includes a step for indicator correction.

The indicator is a dibasic acid which may interfere with the seawater pH measurement. In order to solve the problem, a corrected seawater pH is calculated according to concentration variation of the indicator and pH variation of the mixed solution in dilution so as to realize real-time correction.

Switching the first solenoid valve 4 to the normally open port *b*; switching the fourth solenoid valve 7 to the normally open port *b* when the step of detecting light intensity of the mixed solution and computing absorbance is finished; turning on the peristaltic pump to guide a seawater sample into pipelines to dilute the indicator; continuously measuring a plurality of light intensity of the mixed solution during the course of dilution by the spectrometer and calculating corresponding absorbance and pH, wherein the time required for dilution could be set as 20 to 180 s; due to the fact that the absorbance of the indicator at the isobestic point in the acid state and in the alkali state is merely related to the concentration of the indicator, a rectangular coordinate system is established where the abscissa indicates the absorbance, the ordinate indicates the pH and the measured absorbance and the calculated pH in one-to-one correspondence is denoted as a coordinate point; for a coordinate point where x=0, y indicates the corrected seawater pH where the interference by the indicator is eliminated.

The method further includes a step for flushing pipelines: switching the first solenoid valve 4 to the normally closed port a; switching the fourth solenoid valve 7 to the normally closed port a; reversing the peristaltic pump for 1-10 s; wherein the normally closed port a is connected to the normally closed port a and the seawater sample in the pipelines is flushed out to a position between the fourth solenoid valve 7 and the flow cell and a proper set time for reversing the peristaltic pump could ensure that the sample is completely flushed out but not enter into the flow cell; switching the first solenoid valve 4 to the normally open port b; switching the fourth solenoid valve 7 to the normally open port b; turning on the peristaltic pump to flush the pipelines and the flushing time could be set as 20-180 s.

The present embodiment calibrates the interference by the indicator during the course of the mixture of seawater and indicator. As mentioned above, the indicator is a dibasic acid which may cause a fluctuation of seawater pH when be added into. A correction method of the prior art is to obtain a correction module in lab condition which is then applied to the calculation of seawater pH, and practically the accuracy is far from satisfaction. But the correction method disclosed by the present invention, which is detailed described above could calibrate the interference by the indicator quickly and in situ, and particularly could perform an indicator interference correction for each seawater pH measurement. The method is suitable for in-situ device capable of improve the correction accuracy.

The following example discloses a process for measuring seawater pH during which the seawater pH in-situ measurement device in the above-mentioned embodiment of the present invention, and in the example *thymol* blue is used as the indicator (it should be noted that the present invention is not limited to the indicator). The optical path of the flow cell is 1cm.

The measurement method includes:

Indicator preparation: weighing 0.1954 g of *thymol* blue sodium salt to dissolve in 200 mL high-purity water; adjusting the pH of the solution to $7.9 \pm 0.1$ with 1.0 mol/L hydrochloric acid and sodium hydroxide solution; wherein the concentration of *thymol* blue sodium salt is 2 mmol/L.

Seawater pH determination

    a. switching the first solenoid valve, the second solenoid valve, the third solenoid valve and the fourth solenoid valve to the normally open port *b*; turning on the peristaltic pump to guide a seawater sample to enter and fill with the flow path wherein excess seawater is discharged via the seawater waste liquid discharge pipeline. Seawater sampling is completed.

    b. turning off the peristaltic pump; turning on LEDs; measuring a background light intensity of the seawater sample without being added indicator into in the flow cell by the spectrometer; recording a plurality of light intensity at characteristic wavelengths of *thymol* blue including a wavelength 435 nm at a maximum absorption point in an acid state, a wavelength 596 nm at a maximum absorption point in an alkali state, and a wavelength 494 nm at an isobestic point in an acid state and in an alkali state, which are respectively expressed as $I_{0\lambda 435}$, $I_{0\lambda 596}$ and $I_{0\lambda 494}$.

    c. switching the second solenoid valve to the normally closed port a; switching the third solenoid valve to the normally closed port a; turning on the pulse pump to draw the indicator into the flow path from the indicator bag, wherein excess indicator is discharged through the indicator waste liquid discharge pipeline; turning off the pulse pump when the indicator flows into.

    d. switching the second solenoid valve to the normally open port *b*; switching the third solenoid valve to the normally open port *b*; switching the first solenoid valve to the normally closed port *a*; switching the fourth solenoid valve to the normally closed port *a*; forming a closed loop; turning on the peristaltic pump to fully mix the seawater sample and the indicator and the time for mixing is 60 s; turning off the peristaltic pump.

e. measuring light intensity of the mixed solution in the flow cell and recording light intensity at characteristic wavelengths of *thymol* blue, which are respectively expressed as $I_{\lambda 435}$, $I_{\lambda 596}$ and $I_{\lambda 494}$; computing absorbance by $A_{\lambda} = -\log_{10}(I_{\lambda}/I_{0\lambda})$, which are respectively expressed as $A_{\lambda 435}$, $A_{\lambda 596}$ and $A_{\lambda 494}$.

Calculation of seawater pH

The calculation formula or seawater pH is

$$pH_T = pK_2^T + \log_{10}\frac{[I^{2-}]}{[HI^-]}$$

wherein $pH_T$ represents the pH under the scale of total hydrogen ion, $K_2^T$ is the secondary dissociation constant of *thymol* blue, $[I^{2-}]$ and $[HI^-]$ are the concentrations of thymol blue in the alkali state and in the acid state.

According to Lambert Beer's law, the calculation formula could be written as:

$$pH_T = -\log_{10}(K_2^T) + \log_{10}(\frac{R - e_1}{e_2 - R \times e_3})$$

;

wherein $e_1$, $e_2$ and $e_3$ are ratios of molar absorption coefficients at different forms and different wavelengths of *thymol* blue;

$$e_1 = \frac{\varepsilon_{HI^- \lambda_{596}}}{\varepsilon_{HI^- \lambda_{435}}} , \quad e_2 = \frac{\varepsilon_{I^{2-} \lambda_{596}}}{\varepsilon_{HI^- \lambda_{435}}} , \quad e_3 = \frac{\varepsilon_{I^{2-} \lambda_{435}}}{\varepsilon_{HI^- \lambda_{435}}} , \quad R = \frac{A\lambda_{596}}{A\lambda_{435}},$$

$A_{\lambda 435}$ and $A_{\lambda 596}$ are measured sample absorbance at wavelength 435 nm and at wavelength 596 nm, $\varepsilon_{I2-\lambda 435}$ and $\varepsilon_{I2-\lambda 596}$ are the absorbance coefficients of $I^{2-}$ at wavelength 435 nm and at wavelength 596 nm, $\varepsilon_{HI^-\lambda 435}$ and $\varepsilon_{HI^-\lambda 596}$ are the absorbance coefficients of $HI^-$ at wavelength 435 nm and at wavelength 596 nm. Since the wavelength resolution of the spectrometer used for the measurement in the present embodiment is 1.5 nm, it could directly use the published literature values in the prior art for calculation. The prior art has published the secondary dissociation of *thymol* blue and the constant and molar absorption coefficients, the formula could be referred to are as follows:

$$-\log_{10}(K_2^T) = 4.706 \ S/T + 26.3300 - 7.17218\log T - 0.017316 \times S$$

$$e_1 = -0.00132 + 1.600 \times 10^{-5} \ T$$

$$e_2 = 7.2326 - 0.0299717 \ T + 4.600 \times 10^{-5} \ T^2$$

$e_3 = 0.0223 + 0.0003917 \ T$

The applicable ranges of temperature (T) and salinity (S) are: $278.15 \leq T \leq 308.15K$, $20 \leq S \leq 40$.

The method further includes the following steps for indicator correction and for flushing pipeline:

f. indicator correcting: switching the first solenoid valve to the normally open port *b*; switching the fourth solenoid valve to the normally open port *b*; turning on the peristaltic pump to guide the seawater sample to enter into the pipeline to dilute the indicator *thymol* blue and the dilution time is 60 s; continuously measuring a plurality of light intensity of the mixed solution during the course of dilution by the spectrometer, which are respectively expressed as $I_{i\lambda 435}$, $I_{i\lambda 596}$, $I_{i\lambda 494}$; calculating absorbance correspondingly which are expressed as $A_{i\lambda 435}$, $A_{i\lambda 596}$, $A_{i\lambda 494}$.

Calculating a plurality of mixing solution pHs during the course of the dilution of the indicator *thymol* blue according to the seawater pH calculation formula, which are recorded as pHi; establishing a rectangular coordinate system where the abscissa indicates the absorbance, the ordinate indicates the pH; for a coordinate point where x=0, y indicates the corrected seawater pH that the interference by the indicator *thymol* blue is eliminated.

g. Pipeline flushing: switching the first solenoid valve to the normally closed port *a*; switching the fourth solenoid valve to the normally closed port *a*; reversing the peristaltic pump for 5 s; turning off the peristaltic pump; switching the first solenoid valve to the normally open port *b*; switching the fourth solenoid valve to the normally open port *b*; turning on the peristaltic pump to flush the pipelines and the flushing time is 60 s and the pipeline is cleaned.

**Claims**

1. A high-precision seawater pH in-situ measurement system based on valve terminal, the system including: a flow path module, an optical path module and a circuit module; wherein

   the flow path module includes a valve terminal (3), a flow cell (13), and an indicator bag (1), a pulse pump (2), and a peristaltic pump (11), the indicator bag (1) being connected to the valve terminal (3) through the pulse pump (2), one end of the flow cell (13) being connected to the valve terminal (3) through the peristaltic pump (11), and the other end of the flow cell (13) being connected to the valve terminal (3); the valve terminal (3) being further provided with a seawater sampling pipeline (8), a seawater waste liquid discharge pipeline (9) and an indicator waste liquid discharge pipeline (10);
   the optical path module is configured to measure absorbance of a sample at characteristic wavelengths, the optical path module including a light source coupling module (12), a miniature spectrometer (14) and optical fibers; wherein the light source coupling module (12) includes three LEDs configured to emit light of different wavelengths arranged therein, the light is transmitted through the optical fibers and the flow cell and arrives at the miniature spectrometer (14), and the miniature spectrometer (14) is configured to detect intensity of the light after passing through the flow cell (13);
   the circuit module is respectively connected to the peristaltic pump (11), the pulse pump (2), the valve terminal (3) and the spectrometer (14), the circuit module being configured to control the on-off of the peristaltic pump (11), the pulse pump (2), the valve terminal (3) and the miniature spectrometer (14) and further configured for data collection;
   the valve terminal (3) includes four two-position three-way solenoid valves: a first solenoid valve (4), a second solenoid valve (5), a third solenoid valve (6), and a fourth solenoid valve (7), each of the solenoid valves having a total of three ports: a normally closed port a (3-5), a normally open port b (3-3) and a third port (3-4),
   wherein the seawater sampling pipeline (8) is connected to the normally open port *b* (3-3) of the first solenoid valve (4), the third port (3-4) of the first solenoid valve (4) is connected to the normally open port *b* (3-3) of the second solenoid valve (5), the normally closed port a (3-5) of the second solenoid valve (5) is connected to the indicator bag (1) through the pulse pump (2), the third port (3-4) of the second solenoid valve (5) is connected to the third port (3-4) of the third solenoid valve (6), the normally open port *b* (3-3) of the third solenoid valve (6) is connected to one end of the flow cell (13) through the peristaltic pump (11), the normally closed port a (3-5) of the third solenoid (6) valve is connected to the indicator waste liquid discharge pipeline (10), the normally closed port a (3-5) of the fourth solenoid valve (7) is connected to the normally closed port a (3-5) of the first solenoid valve (4), the normally open port *b* (3-3) of the fourth solenoid valve (7) is connected to the seawater waste liquid discharge pipeline (9), and the third port (3-4) of the fourth solenoid valve (7) is connected to the other end of the flow cell (13).

2. The high-precision seawater pH in-situ measurement system based on valve terminal according to claim 1, wherein the valve terminal (3) includes a base plate (3-1) and a bottom plate (3-2), wherein

   the bottom plate (3-2) is arranged below the base plate (3-1) and the four solenoid valves (4, 5, 6 and 7) are arranged below the bottom plate (3-2);
   the base plate (3-1) and the bottom plate (3-2) are made of acrylic material with high grade of transparency, and are seamlessly bonded by adhesive bonding in a dust-free and clean environment condition; each of the base plate (3-1) and the bottom plate (3-2) is respectively provided with liquid channels therein configured to communicate the solenoid valves with external fluidic pipelines connected to the valve terminal (3);
   the external fluidic pipelines connected to the valve terminal (3) include the fluidic pipeline between the peristaltic pump (11) and the valve terminal (3), the fluidic pipeline between the pulse pump (2) and the valve terminal (3), the indicator waste liquid discharge pipeline (10), the seawater waste liquid discharge pipeline (9), the seawater sampling pipeline (8), the pipeline between the flow cell (13) and the valve terminal (3) and the pipeline between the third port (3-4) of the first solenoid valve (4) and the normally open port b (3-3) of the second solenoid valve (5);
   the liquid channels are further disposed between the first solenoid valve (4) and the fourth solenoid valve (7), and between the second solenoid valve (5) and the third solenoid valve (6);
   the inner diameter of the liquid channels inside the base plate (3-1) and the bottom plate (3-2) is the same as the inner diameter of the external fluidic pipelines connected to the valve terminal (3).

3. The high-precision seawater pH in-situ measurement system based on valve terminal according to claim 1, wherein the flow path is controlled by the valve terminal (3) such that, when performing an in-situ seawater pH measurement, the seawater is first injected into the flow path to determine a background light intensity, then the indicator is injected into the flow path, the flow path is closed into a loop, the seawater and the indicator are mixed within the loop, then the light intensity of the mixed solution is detected for the calculation of the absorbance, and the seawater pH is obtained on the basis of the absorbance.

4. A high-precision seawater pH in-situ measurement method based on valve terminal using the measurement system according to any one of claim 1 to claim 3, the method including:

seawater sampling comprising:
switching the first solenoid valve (4), the second solenoid valve (5), the third solenoid valve (6) and the fourth solenoid valve (7) to the normally open port b (3-3); turning on the peristaltic pump (11) to guide a seawater sample to enter and fill the flow path from the seawater sampling pipeline (8); wherein excess seawater is discharged via the seawater waste liquid discharge pipeline (9);
background light intensity detection comprising:
turning off the peristaltic pump (11); turning on the LEDs; measuring a background light intensity of the seawater sample without indicator by the spectrometer (14) at characteristic wavelengths of selected indicators, wherein the characteristic wavelengths of selected indicators include a wavelength at a maximum absorption point in an acid state, a wavelength at a maximum absorption point in an alkali state, and a wavelength at an isosbestic point in an acid state and in an alkali state, and
wherein the optical signal detected by the spectrometer is expressed as $I_{0\lambda}$ with $\lambda$ indicating the wavelength;
adding indicator comprising:
switching the second solenoid valve (5) to the normally closed port a (3-5); switching the third solenoid valve (6) to the normally closed port a (3-5); turning on the pulse pump (2) to draw a selected indicator into an indicator flow path from the indicator bag (1), wherein excess indicator is discharged through the indicator waste liquid discharge pipeline (10); turning off the pulse pump (2);
sample mixing comprising:
switching the second solenoid valve (5) to the normally open port b (3-3); switching the third solenoid valve (6) to the normally open port b (3-3); switching the first solenoid valve (4) to the normally closed port a (3-5); switching the fourth solenoid valve (7) to the normally closed port a (3-5);
forming a closed loop; turning on the peristaltic pump (11) to fully mix the seawater sample and the indicator, the time for mixing being 20-180 s; turning off the peristaltic pump (11) when the seawater sample and the indicators are fully mixed;
detecting light intensity of the mixed solution and computing absorbance comprising: turning on LEDs when the seawater sample and the indicators are fully mixed; measuring light intensity of the mixed solution wherein the optical signal detected is expressed by $I_\lambda$, computing absorbance by $A_\lambda = -\log_{10}(I_\lambda/I_{0\lambda})$; computing pH on the basis of the absorbance.

5. A high-precision seawater pH in-situ measurement method based on valve terminal according to claim 4, wherein, based on the pH of seawater being determined by the secondary dissociation equilibrium reaction of the acid-base indicator, the calculation formula is:

$$pH_T = pK_2^T + \log_{10}\frac{[I^{2-}]}{[HI^-]}.$$

wherein $pH_T$ represents the pH under the scale of total hydrogen ion, $K_2^T$ is the secondary dissociation constant of the indicator, and $[I^{2-}]$ and $[HI^-]$ are the concentrations of the indicator in the alkali state and in the acid state; the absorbance $A_{\lambda 1}$ of the sample at the wavelength $\lambda_1$ of the maximum absorption point of $[HI^-]$ and the absorbance $A_{\lambda 2}$ of the sample at the wavelength $\lambda_2$ of the maximum absorption point of $[I^{2-}]$, respectively, are measured;
according to Lambert Beer's law, the calculation formula is written as:

$$\mathrm{pH_T} = -\log_{10}(K_2^{\mathrm{T}}) + \log_{10}\left(\frac{R - e_1}{e_2 - R \times e_3}\right)$$

.

wherein $K_2^{\mathrm{T}}$ determines the measurement range of the indicator, and the pH measurement of the seawater sample is carried out by using the applicable seawater pH measurement indicator; wherein

$$e_1 = \frac{\varepsilon_{HI^- \lambda_2}}{\varepsilon_{HI^- \lambda_1}}, \qquad e_2 = \frac{\varepsilon_{I^{2-} \lambda_2}}{\varepsilon_{HI^- \lambda_1}}, \qquad e_3 = \frac{\varepsilon_{I^{2-} \lambda_1}}{\varepsilon_{HI^- \lambda_1}}, \qquad R = \frac{A_{\lambda_2}}{A_{\lambda_1}}$$

wherein $e_1$, $e_2$ and $e_3$ are ratios of molar absorption coefficients at different forms and different wavelengths of the indicator which are related to temperature and salinity; R is the ratio of absorbance; $\varepsilon_{I2-\lambda_1}$ and $\varepsilon_{I2-\lambda_2}$ are the absorbance coefficients of $I^{2-}$ at wavelengths $\lambda_1$ and $\lambda_2$; $\varepsilon_{HI^-\lambda 1}$ and $\varepsilon_{HI^-\lambda 2}$ are the absorbance coefficients of $HI^-$ at $\lambda_1$ and $\lambda_2$.

**6.** A high-precision seawater pH in-situ measurement method based on valve terminal according to claim 4, wherein the method further includes a step for indicator correction comprising: switching the first solenoid valve (4) to the normally open port *b* (3-3); switching the fourth solenoid valve (7) to the normally open port *b* (3-3) when the step of detecting light intensity of the mixed solution and computing absorbance is finished; turning on the peristaltic pump (11) to guide a seawater sample into pipelines to dilute the indicator; continuously measuring a plurality of light intensities of the mixed solution during the course of dilution by the spectrometer (14) and calculating corresponding absorbance and pH, wherein the time required for dilution is set as 20 to 180 s; due to the fact that the absorbance of the indicator at the isosbestic point in the acid state and in the alkali state is merely related to the concentration of the indicator, establishing a rectangular coordinate system where the abscissa indicates the absorbance, the ordinate indicates the pH and the measured absorbance and the calculated pH in one-to-one correspondence is denoted as a coordinate point; and, for a coordinate point where x=0, determining y as an indication of a corrected seawater pH where the interference by the indicator is eliminated.

**Patentansprüche**

**1.** Hochpräzises System zur In-situ-Messung des pH-Werts von Meerwasser auf der Grundlage eines Ventilanschlusses, wobei das System Folgendes umfasst: ein Flusspfadmodul, ein optisches Pfadmodul und ein Schaltungsmodul; wobei

das Flusspfadmodul einen Ventilanschluss (3), eine Flusszelle (13) und einen Indikatorbeutel (1), eine Impulspumpe (2) und eine peristaltische Pumpe (11) umfasst, wobei der Indikatorbeutel (1) mit dem Ventilanschluss (3) durch die Impulspumpe (2) verbunden ist, ein Ende der Flusszelle (13) mit dem Ventilanschluss (3) durch die peristaltische Pumpe (11) verbunden ist und das andere Ende der Flusszelle (13) mit dem Ventilanschluss (3) verbunden ist; das Ventilterminal (3) ist ferner mit einer Meerwasser-Probenahmeleitung (8), einer Meerwasser-Abwasserabflussleitung (9) und einer Indikator-Abwasserabflussleitung (10) versehen; das optische Pfadmodul so konfiguriert ist, dass es die Absorption einer Probe bei charakteristischen Wellenlängen misst, wobei das Modul für den optischen Pfad ein Lichtquellen-Kopplungsmodul (12), ein Miniaturspektrometer (14) und optische Fasern umfasst; wobei das Lichtquellen-Kopplungsmodul (12) drei LEDs umfasst, die so konfiguriert sind, dass sie Licht unterschiedlicher Wellenlängen emittieren, das Licht durch die optischen Fasern und die Flusszelle übertragen wird und am Miniaturspektrometer (14) ankommt, und das Miniaturspektrometer (14) so konfiguriert ist, dass es die Intensität des Lichts erfasst, nachdem es die Flusszelle (13) passiert hat; das Schaltungsmodul jeweils mit der Schlauchpumpe (11), der Impulspumpe (2), der Ventilinsel (3) und dem Spektrometer (14) verbunden ist, wobei das Schaltungsmodul so konfiguriert ist, dass es das Ein- und Ausschalten der Schlauchpumpe (11), der Impulspumpe (2), der Ventilinsel (3) und des Miniaturspektrometers (14) steuert, und ferner zur Datenerfassung konfiguriert ist; die Ventilinsel (3) vier Zwei-Positionen-Drei-Wege-Magnetventile umfasst: ein erstes Magnetventil (4), ein zweites Magnetventil (5), ein drittes Magnetventil (6) und ein viertes Magnetventil (7), wobei jedes der Magnetventile insgesamt drei Anschlüsse aufweist: einen normalerweise geschlossenen Anschluss *a* (3-5), einen normalerweise offenen Anschluss b (3-3) und einen dritten Anschluss (3-4), wobei die Meerwasser-Probenahmeleitung (8) mit dem normalerweise offenen Anschluss b (3-3) des ersten Magnetventils (4) verbunden ist, der dritte Anschluss (3-4) des ersten Magnetventils (4) mit dem normalerweise offenen Anschluss *b* (3-3) des zweiten Magnetventils (5) verbunden ist, der normalerweise geschlossene Anschluss *a* (3-5) des zweiten Magnetventils (5) über die Impulspumpe (2) mit dem Anzeigebeutel (1) verbunden ist, der dritte Anschluss (3-4) des zweiten Magnetventils (5) ist mit dem dritten Anschluss (3-4) des dritten Magnetventils (6) verbunden, der normalerweise offene Anschluss *b* (3-3) des dritten Magnetventils (6) ist über die peristaltische Pumpe (11) mit einem Ende der Durchflusszelle (13) verbunden, der normalerweise geschlossene Anschluss *a* (3-5) des dritten Magnetventils (6) ist mit der Abflussleitung (10) für die Anzeigeflüssigkeit verbunden, der normalerweise geschlossene Anschluss *a* (3-5) des vierten Magnetventils (7) ist mit dem normalerweise geschlossenen Anschluss *a* (3-5) des ersten Magnetventils (4) verbunden, der normalerweise offene Anschluss b (3-3) des vierten Magnetventils (7) ist mit der Seewasserabflussleitung (9) verbunden, und der dritte Anschluss (3-4) des vierten Magnetventils (7) ist mit dem anderen Ende der Durchflusszelle (13) verbunden.

2. Hochpräzises System zur In-situ-Messung des pH-Werts von Meerwasser auf der Grundlage eines Ventilanschlusses nach Anspruch 1, wobei der Ventilanschluss (3) eine Grundplatte (3-1) und eine Bodenplatte (3-2) aufweist, wobei die Bodenplatte (3-2) unterhalb der Grundplatte (3-1) und die vier Magnetventile (4, 5, 6 und 7) unterhalb der Bodenplatte (3-2) angeordnet sind;

die Grundplatte (3-1) und die Bodenplatte (3-2) aus Acrylmaterial mit hoher Transparenz hergestellt sind und nahtlos durch Kleben in einer staubfreien und sauberen Umgebung verbunden sind; jede der Grundplatte (3-1) und der Bodenplatte (3-2) jeweils mit Flüssigkeitskanälen darin versehen ist, die so konfiguriert sind, dass sie die Magnetventile mit externen Fluidleitungen verbinden, die mit der Ventilinsel (3) verbunden sind;
die externen Fluidleitungen, die mit dem Ventilanschluss (3) verbunden sind, umfassen die Fluidleitung zwischen der peristaltischen Pumpe (11) und dem Ventilanschluss (3), die Fluidleitung zwischen der Impulspumpe (2) und dem Ventilanschluss (3), die Abwasserabflussleitung (10) des Indikators, die Abflussleitung (10) für die Anzeigeflüssigkeit, die Abflussleitung (9) für das Meerwasser, die Leitung (8) für die Meerwasserprobenahme, die Leitung zwischen der Durchflusszelle (13) und dem Ventilanschluss (3) und die Leitung zwischen dem dritten Anschluss (3-4) des ersten Magnetventils (4) und dem normalerweise offenen Anschluss *b* (3-3) des zweiten Magnetventils (5);
die Flüssigkeitskanäle sind außerdem zwischen dem ersten Magnetventil (4) und dem vierten Magnetventil (7) und zwischen dem zweiten Magnetventil (5) und dem dritten Magnetventil (6) angeordnet;
der Innendurchmesser der Flüssigkeitskanäle im Inneren der Grundplatte (3-1) und der Bodenplatte (3-2) dem Innendurchmesser der mit der Ventilinsel (3) verbundenen externen Fluidleitungen entspricht.

3. Hochpräzises System zur In-situ-Messung des pH-Werts von Meerwasser auf der Grundlage eines Ventilanschlusses nach Anspruch 1, wobei der Flusspfad durch den Ventilanschluss (3) so gesteuert wird, dass bei der Durchführung einer In-situ-Messung des pH-Werts von Meerwasser zunächst das Meerwasser in den Flusspfad injiziert wird, um eine Hintergrundlichtintensität zu bestimmen, dann der Indikator in den Flusspfad injiziert wird, der Strömungsweg zu einer Schleife geschlossen wird, das Meerwasser und der Indikator innerhalb der Schleife gemischt werden, dann die Lichtintensität der gemischten Lösung für die Berechnung der Absorption erfasst wird und der Meerwasser-pH auf der Grundlage der Absorption erhalten wird.

4. Hochpräzises System zur In-situ-Messung des pH-Werts von Meerwasser auf der Grundlage eines Ventilanschlusses nach einem der Ansprüche 1 bis 3, wobei das Verfahren umfasst:

Probenahme von Meerwasser, umfassend: Umschalten des ersten Magnetventils (4), des zweiten Magnetventils (5), des dritten Magnetventils (6) und des vierten Magnetventils (7) auf den normalerweise offenen Anschluss *b* (3-3); Einschalten der peristaltischen Pumpe (11), um eine Meerwasserprobe in den Strömungsweg von der Meerwasserprobenahmeleitung (8) einzubringen und diesen zu füllen; wobei überschüssiges Meerwasser über die Meerwasserabflussleitung (9) abgeleitet wird;
Erkennung der Hintergrundlichtintensität mit folgenden Schritten: Abschalten der peristaltischen Pumpe (11); Einschalten der LEDs; Messen einer Hintergrundlichtintensität der Meerwasserprobe ohne Indikator durch das Spektrometer (14) bei charakteristischen Wellenlängen ausgewählter Indikatoren, wobei die charakteristischen Wellenlängen ausgewählter Indikatoren eine Wellenlänge bei einem maximalen Absorptionspunkt in einem sauren Zustand, eine Wellenlänge bei einem maximalen Absorptionspunkt in einem alkalischen Zustand und eine Wellenlänge bei einem isosbestischen Punkt in einem sauren Zustand und in einem alkalischen Zustand umfassen, und wobei das durch das Spektrometer erfasste optische Signal als $I_{0\lambda}$ ausgedrückt wird, wobei $\lambda$ die Wellenlänge angibt;

Hinzufügen eines Indikators, umfassend: Schalten des zweiten Magnetventils (5) auf den normalerweise geschlossenen Anschluss *a* (3-5); Schalten des dritten Magnetventils (6) auf den normalerweise geschlossenen Anschluss *a* (3-5); Einschalten der Impulspumpe (2), um einen ausgewählten Indikator in einen Indikatorströmungspfad aus dem Indikatorbeutel (1) zu ziehen, wobei überschüssiger Indikator durch die Indikatorabfallflüssigkeitsabflussleitung (10) abgeleitet wird; Abschalten der Impulspumpe (2);

Probenmischung, umfassend: Schalten des zweiten Magnetventils (5) auf den normalerweise offenen Anschluss *b* (3-3); Schalten des dritten Magnetventils (6) auf den normalerweise offenen Anschluss *b* (3-3); Schalten des ersten Magnetventils (4) auf den normalerweise geschlossenen Anschluss *a* (3-5); Schalten des vierten Magnetventils (7) auf den normalerweise geschlossenen Anschluss *a* (3-5); Bilden einer geschlossenen Schleife; Einschalten der peristaltischen Pumpe (11), um die Meerwasserprobe und den Indikator vollständig zu mischen, wobei die Zeit für das Mischen 20-180 s beträgt; Abschalten der peristaltischen Pumpe (11), wenn die Meerwasserprobe und die Indikatoren vollständig gemischt sind;

Erfassen der Lichtintensität der gemischten Lösung und Berechnen der Absorption, umfassend: Einschalten von LEDs, wenn die Meerwasserprobe und die Indikatoren vollständig gemischt sind; Messen der Lichtintensität der gemischten Lösung, wobei das erfasste optische Signal durch $I_\lambda$ ausgedrückt wird; Berechnen der Absorption durch $A_\lambda = -\log_{10}(I/I_{0\lambda})$; Berechnen des pH-Wertes auf der Grundlage der Absorption.

5. Hochpräzises System zur In-situ-Messung des pH-Werts von Meerwasser auf der Grundlage eines Ventilanschlusses nach Anspruch 4, wobei auf der Grundlage des pH-Werts des Meerwassers durch die sekundäre Dissoziationsgleichgewichtsreaktion des Säure-Base-Indikators bestimmt wird, die Berechnungsformel lautet:

$$pH_T = pK_2^T + \log_{10}\frac{[I^{2-}]}{[HI^-]},$$

wobei $pH_T$ den pH-Wert nach der Gesamtwasserstoffionenskala darstellt, $K_2^T$ die sekundäre Dissoziationskonstante des Indikators ist und $[I^{2-}]$ und $[HI^-]$ die Konzentrationen des Indikators im alkalischen Zustand und im sauren Zustand sind;

wird die Absorption $A_{\lambda 1}$ der Probe bei der Wellenlänge $\lambda_1$ des maximalen Absorptionspunktes von $[HI^-]$ bzw. die Absorption $A_{\lambda 2}$ der Probe bei der Wellenlänge $\lambda_2$ des maximalen Absorptionspunktes von $[I^{2-}]$ gemessen; nach dem Lambert-Beer'schen Gesetz wird die Berechnungsformel wie folgt geschrieben

$$pH_T = -\log_{10}(K_2^T) + \log_{10}\left(\frac{R - e_1}{e_2 - R \times e_3}\right),$$

wobei $K_2^T$ den Messbereich des Indikators bestimmt, und die pH-Messung der Meerwasserprobe unter Verwendung des anwendbaren Meerwasser-pH-Messindikators durchgeführt wird; wobei

$$e_1 = \frac{\varepsilon_{HI^- \lambda_2}}{\varepsilon_{HI^- \lambda_1}}, \qquad e_2 = \frac{\varepsilon_{I^{2-} \lambda_2}}{\varepsilon_{HI^- \lambda_1}}, \qquad e_3 = \frac{\varepsilon_{I^{2-} \lambda_1}}{\varepsilon_{HI^- \lambda_1}}, \qquad R = \frac{A_{\lambda_2}}{A_{\lambda_1}}$$

wobei $e_1$, $e_2$ und $e_3$ Verhältnisse der molaren Absorptionskoeffizienten bei verschiedenen Formen und verschiedenen Wellenlängen des Indikators sind, die mit der Temperatur und dem Salzgehalt zusammenhängen; R das Verhältnis der Absorption ist; $\varepsilon_{I2-\lambda_1}$ und $\varepsilon_{I2-\lambda_2}$ die Absorptionskoeffizienten von $I^{2-}$ bei den Wellenlängen $\lambda_1$ und $\lambda_2$ sind; $\varepsilon_{HI^-\lambda_1}$ und $\varepsilon_{HI^-\lambda_2}$ sind die Absorptionskoeffizienten von $HI^-$ bei $\lambda_1$ und $\lambda_2$.

6. Hochpräzises Meerwasser-pH-in-situ-Messverfahren auf der Basis eines Ventilanschlusses nach Anspruch 4, wobei das Verfahren ferner einen Schritt zur Indikatorkorrektur umfasst, der Folgendes umfasst: Schalten des ersten Magnetventils (4) auf den normalerweise offenen Anschluss *b* (3-3); Schalten des vierten Magnetventils (7) auf den normalerweise offenen Anschluss *b* (3-3), wenn der Schritt des Erfassens der Lichtintensität der gemischten Lösung und des Berechnens der Absorption beendet ist; Einschalten der peristaltischen Pumpe (11), um eine Meerwasserprobe in Rohrleitungen zu leiten, um den Indikator zu verdünnen; kontinuierliches Messen einer Vielzahl von Lichtintensitäten der gemischten Lösung während des Verlaufs der Verdünnung durch das Spektrometer (14) und Berechnen der entsprechenden Absorption und des pH-Werts, wobei die für die Verdünnung erforderliche Zeit auf 20 bis 180 s eingestellt ist; aufgrund der Tatsache, daß die Absorption des Indikators am isosbestischen Punkt im sauren Zustand und im alkalischen Zustand lediglich mit der Konzentration des Indikators zusammenhängt, Aufstellen eines rechtwinkligen Koordinatensystems, in dem die Abszisse die Absorption angibt, die Ordinate den pH-Wert angibt und die gemessene Absorption und der berechnete pH-Wert in Eins-zu-Eins-Entsprechung als ein Koordinatenpunkt bezeichnet werden; und für einen Koordinatenpunkt, an dem x=0 ist, Angeben von y als eine Angabe eines korrigierten Meerwasser-pH-Wertes, bei dem die Störung durch den Indikator beseitigt ist.

**Revendications**

1. Système de mesure in situ de haute précision du pH d'eau de mer fondé sur un terminal de vanne, le système comprenant : un module de trajet d'écoulement, un module de trajet optique et un module de circuit ; dans lequel

   le module de trajet d'écoulement comprend un terminal de vanne (3), une cellule d'écoulement (13), une poche d'indicateur (1), une pompe à impulsion (2) et une pompe péristaltique (11), la poche d'indicateur (1) étant reliée au terminal de vanne (3) par la pompe à impulsion (2), une extrémité de la cellule d'écoulement (13) étant reliée au terminal de vanne (3) par la pompe péristaltique (11), et l'autre extrémité de la cellule d'écoulement (13) étant reliée au terminal de vanne (3) ; le terminal de vanne (3) est en outre équipé d'une conduite d'échantillonnage d'eau de mer (8), d'une conduite d'évacuation de liquide résiduaire d'eau de mer (9) et d'une conduite d'évacuation de liquide résiduaire d'indicateur (10) ;
   le module de chemin optique est configuré pour mesurer l'absorbance d'un échantillon à des longueurs d'onde caractéristiques,
   le module de chemin optique comprend un module de couplage de source lumineuse (12), un spectromètre miniature (14) et des fibres optiques ; dans lequel le module de couplage de source lumineuse (12) comprend trois DEL configurées pour émettre de la lumière de différentes longueurs d'onde disposées à l'intérieur de celui-ci, la lumière est transmise à travers les fibres optiques et la cellule d'écoulement et arrive au spectromètre miniature (14), et le spectromètre miniature (14) est configuré pour détecter l'intensité de la lumière après avoir traversé la cellule d'écoulement (13) ;
   le module de circuit est respectivement relié à la pompe péristaltique (11), à la pompe à impulsions (2), au terminal de vanne (3) et au spectromètre (14), le module de circuit étant configuré pour commander la mise en marche et l'arrêt de la pompe péristaltique (11), de la pompe à impulsions (2), du terminal de vanne (3) et du spectromètre miniature (14), et étant en outre configuré pour collecter des données ;
   le terminal de vanne (3) comprend quatre électrovannes à trois voies à deux positions : une première électrovanne (4), une deuxième électrovanne (5), une troisième électrovanne (6) et une quatrième électrovanne (7), chacune des électrovannes ayant un total de trois orifices : un orifice normalement fermé *a* (3-5), un orifice normalement ouvert *b* (3-3) et un troisième orifice (3-4),
   dans lequel la conduite d'échantillonnage d'eau de mer (8) est reliée à l'orifice normalement ouvert *b* (3-3) de la première électrovanne (4), le troisième orifice (3-4) de la première électrovanne (4) est relié à l'orifice normalement ouvert *b* (3-3) de la deuxième électrovanne (5), l'orifice normalement fermé *a* (3-5) de la deuxième électrovanne (5) est relié à la poche d'indicateur (1) par l'intermédiaire de la pompe à impulsions (2), le troisième orifice (3-4) de la deuxième électrovanne (5) est relié au troisième orifice (3-4) de la troisième électrovanne (6), l'orifice normalement ouvert b (3-3) de la troisième électrovanne (6) est relié à une extrémité de la cellule d'écoulement (13) par l'intermédiaire de la pompe péristaltique (11), l'orifice normalement fermé *a* (3-5) de la troisième électrovanne (6) est relié à la conduite d'évacuation de liquide résiduaire d'indicateur (10), l'orifice normalement fermé *a* (3-5) de la quatrième électrovanne (7) est relié à l'orifice normalement fermé *a* (3-5) de la première électrovanne (4), l'orifice normalement ouvert *b* (3-3) de la quatrième électrovanne (7) est relié à la conduite d'évacuation de liquide résiduaire d'eau de mer (9), et le troisième orifice (3-4) de la quatrième électrovanne (7) est relié à l'autre extrémité de la cellule d'écoulement (13).

2. Système de mesure in situ de haute précision du pH d'eau de mer fondé sur un terminal de vanne selon la revendication 1, dans lequel le terminal de vanne (3) comprend une plaque de base (3-1) et une plaque inférieure (3-2), dans lequel la plaque inférieure (3-2) est disposée sous la plaque de base (3-1) et les quatre électrovannes (4, 5, 6 et 7) sont disposées sous la plaque inférieure (3-2) ;

   la plaque de base (3-1) et la plaque inférieure (3-2) sont fabriquées en matériau acrylique avec un haut degré de transparence, et sont collées sans soudure par collage dans un environnement propre et exempt de poussière ; la plaque de base (3-1) et la plaque inférieur (3-2) sont respectivement pourvues de canaux de liquide configurés pour faire communiquer les électrovannes avec des conduites de fluide externes reliées au terminal de vanne (3) ;

les conduites fluidiques externes reliées au terminal de vanne (3) comprennent la conduite fluidique entre la pompe péristaltique (11) et le terminal de vanne (3), la conduite fluidique entre la pompe à impulsions (2) et le terminal de vanne (3), la conduite d'évacuation de liquide résiduaire d'indicateur (10), la conduite d'évacuation de liquide résiduaire d'eau de mer (9), la conduite d'échantillonnage d'eau de mer (8), la conduite entre la cellule d'écoulement (13) et le terminal de vanne (3) et la conduite entre le troisième orifice (3-4) de la première électrovanne (4) et l'orifice normalement ouvert *b* (3-3) de la deuxième électrovanne (5) ;

les canaux de liquide sont en outre disposés entre la première électrovanne (4) et la quatrième électrovanne (7), et entre la deuxième électrovanne (5) et la troisième électrovanne (6) ;

le diamètre intérieur des canaux de liquide à l'intérieur de la plaque de base (3-1) et de la plaque de fond (3-2) est le même que le diamètre intérieur des conduites de fluide externes reliées au terminal de vanne (3).

3. Système de mesure in situ de haute précision du pH d'eau de mer fondé sur un terminal de vanne selon la revendication 1, dans lequel le trajet d'écoulement est contrôlé par le terminal de vanne (3) de telle sorte que, lors d'une mesure in-situ du pH d'eau de mer, l'eau de mer est d'abord injectée dans le trajet d'écoulement afin de déterminer une intensité lumineuse de fond, l'indicateur est ensuite injecté dans le trajet d'écoulement, le trajet d'écoulement est fermé en boucle, l'eau de mer et l'indicateur sont mélangés dans la boucle, l'intensité lumineuse de la solution mélangée est ensuite détectée pour le calcul de l'absorbance, et le pH d'eau de mer est obtenu sur la base de l'absorbance.

4. Procédé de mesure in situ de haute précision du pH d'eau de mer fondé sur un terminal de vanne utilisant le système de mesure selon l'une quelconque des revendications 1 à 3, le procédé comprenant les étapes consistant à :

échantillonner de l'eau de mer comprenant : la commutation de la première électrovanne (4), de la deuxième électrovanne (5), de la troisième électrovanne (6) et de la quatrième électrovanne (7) sur l'orifice normalement ouvert *b* (3-3) ; la mise en marche de la pompe péristaltique (11) pour guider un échantillon d'eau de mer afin qu'il entre et remplisse le trajet d'écoulement à partir de la conduite d'échantillonnage d'eau de mer (8) ; dans lequel l'eau de mer excédentaire étant évacué par la conduite d'évacuation de liquide résiduaire d'eau de mer (9) ;

détecter l'intensité lumineuse de fond comprenant : l'arrêt de la pompe péristaltique (11) ; l'allumage des DEL ; la mesure d'une intensité lumineuse de fond de l'échantillon d'eau de mer sans indicateur par le spectromètre (14) à des longueurs d'onde caractéristiques d'indicateurs sélectionnés, dans lequel les longueurs d'onde caractéristiques d'indicateurs sélectionnés comprennent une longueur d'onde à un point d'absorption maximale dans un état acide, une longueur d'onde à un point d'absorption maximale à l'état alcalin, et une longueur d'onde à un point isosbestic à l'état acide et à l'état alcalin, et dans lequel le signal optique détecté par le spectromètre est exprimé par $I_{0\lambda}$, $\lambda$ indiquant la longueur d'onde ;

ajouter un indicateur comprenant : la commutation de la deuxième électrovanne (5) sur l'orifice normalement fermé *a* (3-5); la commutation de la troisième électrovanne (6) sur l'orifice normalement fermé *a* (3-5); la mise en marche de la pompe à impulsions (2) pour aspirer un indicateur sélectionné dans un trajet d'écoulement d'indicateur à partir de la poche d'indicateur (1), dans lequel l'indicateur excédentaire est évacué par la conduite d'évacuation de liquide résiduaire d'indicateur (10) ; l'arrêt de la pompe à impulsions (2) ;

mélanger des échantillons comprenant : la commutation de la deuxième électrovanne (5) sur l'orifice normalement ouvert *b* (3-3) ; la commutation de la troisième électrovanne (6) sur l'orifice normalement ouvert *b* (3-3) ; la commutation de la première électrovanne (4) sur l'orifice normalement fermé *a* (3-5) ; la commutation de la quatrième électrovanne (7) sur l'orifice normalement fermé *a* (3-5) ; la formation d'une boucle fermée ; la mise en marche de la pompe péristaltique (11) pour mélanger complètement l'échantillon d'eau de mer et l'indicateur, la durée du mélange étant comprise entre 20 et 180 s ; l'arrêt de la pompe péristaltique (11) lorsque l'échantillon d'eau de mer et les indicateurs sont complètement mélangés ;

détecter l'intensité lumineuse de la solution mélangée et calculer l'absorbance comprenant : l'allumage des DEL lorsque l'échantillon d'eau de mer et les indicateurs sont complètement mélangés ; la mesure de l'intensité lumineuse de la solution mélangée où le signal optique détecté est exprimé par $I_A$, le calcul de l'absorbance par $A_\lambda = -\log_{10}(I_\lambda / I_{0\lambda})$ ; le calcul du pH sur la base de l'absorbance.

5. Procédé de mesure in situ de haute précision du pH d'eau de mer fondé sur un terminal de vanne selon la revendication 4, dans lequel, sur la base du pH de l'eau de mer déterminé par la réaction d'équilibre de dissociation secondaire de l'indicateur acido-basique, la formule de calcul est la suivante :

$$pH_T = pK_2^T + \log_{10} \frac{[I^{2-}]}{[HI^-]},$$

où

$pH_T$ représente le pH sous l'échelle de l'ion hydrogène total, $K_2^T$ est la constante de dissociation secondaire de l'indicateur, et

$[I^{2-}]$ et $[HI^-]$ sont les concentrations de l'indicateur à l'état alcalin et à l'état acide ;

l'absorbance $A_{\lambda 1}$ de l'échantillon à la longueur d'onde $\lambda_1$ du point d'absorption maximale de $[HI^-]$ et l'absorbance $A_{\lambda 2}$ de l'échantillon à la longueur d'onde $\lambda_2$ du point d'absorption maximale de $[I^{2-}]$, respectivement, sont mesurées ;

selon la loi de Lambert Beer, la formule de calcul s'écrit comme suit :

$$pH_T = -\log_{10}(K_2^T) + \log_{10}\left(\frac{R - e_1}{e_2 - R \times e_3}\right),$$

dans lequel $K_2^T$ détermine la plage de mesure de l'indicateur, et la mesure de pH de l'échantillon d'eau de mer est effectuée à l'aide de l'indicateur de mesure de pH d'eau de mer applicable ; dans lequel

$$e_1 = \frac{\varepsilon_{HI^--\lambda_2}}{\varepsilon_{HI^--\lambda_1}}, \qquad e_2 = \frac{\varepsilon_{I^{2-}-\lambda_2}}{\varepsilon_{HI^--\lambda_1}}, \qquad e_3 = \frac{\varepsilon_{I^{2-}-\lambda_1}}{\varepsilon_{HI^--\lambda_1}}, \qquad R = \frac{A_{\lambda_2}}{A_{\lambda_1}}$$

où $e_1$, $e_2$ et $e_3$ sont les rapports des coefficients d'absorption molaire sous différentes formes et à différentes longueurs d'onde de l'indicateur, qui sont liés à la température et à la salinité ; R est le rapport d'absorbance ; $\varepsilon_{I2-\lambda_1}$ et $\varepsilon_{I2-\lambda_2}$ sont les coefficients d'absorbance de $I^{2-}$ aux longueurs d'onde $\lambda_1$ et $\lambda_2$; $\varepsilon_{HI^--\lambda_1}$ et $\varepsilon_{HI^--\lambda_2}$ sont les coefficients d'absorbance de $HI^-$ aux longueurs d'onde $\lambda_1$ et $\lambda_2$.

6. Procédé de mesure in situ de haute précision du pH d'eau de mer fondé sur un terminal de vanne selon la revendication 4, dans lequel le procédé comprend en outre une étape de correction d'indicateur comprenant : la commutation de la première électrovanne (4) sur l'orifice normalement ouvert *b* (3-3) ; la commutation de la quatrième électrovanne (7) sur l'orifice normalement ouvert *b* (3-3) lorsque l'étape de détection d'intensité lumineuse de la solution mélangée et de calcul d'absorbance est terminée ; la mise en marche de la pompe péristaltique (11) pour guider un échantillon d'eau de mer dans les conduites afin de diluer l'indicateur ; la mesure en continu d'une pluralité d'intensités lumineuses de la solution mélangée au cours de la dilution par le spectromètre (14) et le calcule de l'absorbance et du pH correspondants, dans lequel le temps nécessaire à la dilution étant compris entre 20 et 180 s ; étant donné que l'absorbance de l'indicateur au point isostatique à l'état acide et à l'état alcalin est simplement liée à la concentration de l'indicateur, l'établissement d'un système de coordonnées rectangulaires dans lequel l'abscisse indique l'absorbance, l'ordonnée indique le pH et l'absorbance mesurée et le pH calculé en correspondance biunivoque sont désignés comme point de coordonnées ; et, pour un point de coordonnées où x=0, la détermination de y comme indication d'un pH corrigé d'eau de mer où l'interférence de l'indicateur est éliminée.

Fig.1

Fig.2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 103698287 A
- CN 105241829 A
- CN 103439258 A

### Non-patent literature cited in the description

- **SPAULDING REGGIE S. et al.** Autonomous in Situ Measurements of Seawater Alkalinity. *ENVIRONMENTAL SCIENCE & TECHNOLOGY, US,* 22 July 2014, vol. 48 (16), ISSN 0013-936X, 9573-9581
- **RÉROLLE VICTOIRE M.C. et al.** Seawater-pH measurements for ocean-acidification observations. *TRAC TRENDS IN ANALYTICAL CHEMISTRY, AMSTERDAM, NL,* 2012, vol. 40, ISSN 0165-9936, 146-157